# EUROPEAN PATENT SPECIFICATION

(11) **EP 2 931 303 B1**
(45) Date of publication and mention of the grant of the patent: **19.07.2017**
(21) Application number: 13826955.0
(22) Date of filing: 16.12.2013
(51) Int. Cl.: A61K 38/40, C07K 14/79, A61P 15/04

(54) **LACTOFERRIN FOR USE IN THE TREATMENT OF PRETERM DELIVERY AND MISCARRIAGE**
LACTOFERRIN ZUR BEHANDLUNG VON FRÜHGEBURT UND FEHLGEBURT
LACTOFERRIN POUR LE TRAITEMENT DE LA PRÉMATURITEÉ ET LA FAUSSE COUCHE

(30) Priority: 17.12.2012 IT MI20122152
(43) Date of publication of application: 21.10.2015
(73) Proprietor: Progine Farmaceutici S.r.l., 50040 Calenzano (FI) (IT)
(72) Inventor: LANDI, Lapo, I-50040 Calenzano FI (IT)
(74) Representative: Asensio, Raffaella Consuelo
(86) International application number: PCT/IB2013/060994
(87) International publication number: WO 2014/097123

(56) References cited:
- WO-A1-2006/098625
- WO-A1-2012/084459
- WO-A2-2011/150393
- IT-A1- BO20 080 619
- ANTONIO CIANCI ET AL: "Influence of lactoferrin in preventing preterm delivery: A pilot study", MOLECULAR MEDICINE REPORTS, vol. 5, no. 1, 13 September 2011 (2011-09-13), pages 162-166, XP055056961, ISSN: 1791-2997, DOI: 10.3892/mmr.2011.584
- YAKUWA K ET AL: "Recombinant human lactoferrin has a potential to suppresses uterine cervical ripening in preterm delivery in animal model", ARCHIVES OF GYNECOLOGY AND OBSTETRICS, SPRINGER, BERLIN, DE, vol. 275, no. 5, May 2007 (2007-05), pages 331-334, XP019491216, ISSN: 1432-0711, DOI: 10.1007/S00404-006-0261-9 [retrieved on 2006-10-10]

## Description

The present invention relates to a composition comprising an high dosage of lactoferrin and to its use in the prevention of miscarriage and/or preterm delivery. Lactoferrin is a polypeptide with a molecular weight of approximately 80 kDa, which is a member of the transferrin protein family.

Lactoferrin coordinates the ferric ion (Fe³⁺) by two binding sites. Normally it has a ferric content that is variable, but always below the maximum possible degree of saturation.

Lactoferrin is found mainly in milk, but it is present in many mucosal secretions, such as tears and saliva.

The antimicrobial activity of lactoferrin is related to its affinity for Fe³⁺.

The combination of lactoferrin with ferric ions in the mucous secretions modulates the activity and the ability of bacteria and viruses to adhere to the cell membranes, since lactoferrin subtracts from the environment the iron necessary for bacterial cell replication. In fact, some bacteria require iron in order to perform cell replication and lactoferrin subtracts iron from the surrounding environment, preventing the proliferation of said bacteria.

Lactoferrin is normally contained in neutrophil granulocytes. In humans, the gene coding for lactoferrin is located on chromosome 3 with location 3q21 - q23. Lactoferrin also has a bactericidal activity independent of its ability to bind iron ions, since it is able to attack and to break the bacterial membrane by lysis, exploiting the affinity of its cationic domains for the negatively charged bacterial membrane. This activity, in combination with lysozyme, an enzyme capable of cleaving the β1-4 glycosidic bonds of peptidoglycan, results in cytolysis of the bacterium.

The use of lactoferrin has been reported as an antibacterial (WO9806425) agent, for the treatment of anemias (WO2004060392), as a component of infant food formulas (WO2012091945 and WO2010130643), as a livestock supplement (WO03047363) and in the gynecological area (IT1392620).

One of the disadvantages of lactoferrin is its prevailing hepatic metabolism, which causes elimination of 99% of the molecule from the body within 24 h from administration.

In view of this aspect, according to the teachings in the prior art, the administration of lactoferrin should be carried out repeatedly during the day every 4 hours (or less) with single low amount dosages. For example, in WO2012084459 lactoferrin is repeatedly administered via intravaginal route in single 100 mg unit dosages every 4, 6, 8 or 12 hours depending on the frequency of uterine contractions (in combination with oral therapy).

This dosage regimen is clearly inconvenient, especially when the administration is to be carried out topically (e.g. intravaginally), because the subject is thus forced to repeat the intake several times over a 24-hour period, including at night and during normal daytime activities. This disadvantage can make it difficult to comply with the prescribed regimen and may eventually expose the subject to the risk of miscarriage or preterm delivery. Preterm delivery is defined as simultaneous presence of contractions (more than 6 contractions in 1 hour), pain and cervical changes, either shortening and/or softening or dilatation by examination (manual or by ultrasound scanning) earlier than the term generally accepted as safe by physicians in the normal hospital practice. Interventions to prevent pre-term labour (PTL) and delivery, including bed rest, antibiotics, cervical cerclage etc, have been largely unsuccessful (Berghella et al., J Obstet Gynecol 2011, 117 : 663 - 671). Furthermore, more common therapies (i.e. beta mimetics, atosiban, calcium channel blockers) are not always successful (Locci et al. J Obstet Gynecol 2006, 26: 396-401). This failure is likely to be due to the multifactorial aetiology of PTL.

One aim of the present disclosure is a method for the treatment and prevention of preterm birth and miscarriage, which is effective and allows easy compliance to the prescribed regimen.

A task of the present invention is to provide a formulation that allows easy and effective administration of lactoferrin for the prevention and treatment of preterm births and of spontaneous miscarriage.

In accordance with the present invention, these aims, and others that will be more evident hereinafter, are achieved by means of lactoferrin for use in the prevention and treatment of preterm delivery and miscarriage, wherein said use comprises administering a composition, wherein the amount of lactoferrin is from 250 to 800 mg per unit, once or twice a day by topical intravaginal administration to a subject in need thereof. These objects have also been achieved by a composition in a unit dosage form suitable for single intravaginal administration comprising at least one excipient suitable for intravaginal intake and an amount of lactoferrin from 350 to 800 mg of lactoferrin.

If not otherwise specified, within the scope of the present invention the percentages are referred to the weight of a component on the total weight of the composition.

As used herein, the term "comprises", referred to a component of a composition, is understood to encompass also the possibility that said component constitutes 100% of the composition, i.e. a mixture "comprising" component A may also consist of 100% of A.

Within the scope of the present invention, the content of lactoferrin comprised in a single composition/unit dosage form indicates the whole weight of lactoferrin in the composition/unit form. For example, a composition comprising 800 mg of lactoferrin and at least one excipient contains 800 mg of lactoferrin, together with additional component(s), which are different from lactoferrin.

Within the scope of the present invention, the term "lactoferrin" indicates either the naturally occurring, synthetic or semi-synthetic complete protein or a biologically active fragment thereof, as well as their polymorphic forms. As non-limiting examples, lactoferrin for use in the present invention can be of human origin or of bovine origin.

The term "topical administration" refers to local administration of lactoferrin in the mucosa of the vaginal cavity, for example by application of a vaginal tablet, a cream, an ointment, a pessary, a suppository or similar dosage forms, excluding parenteral administration.

Within the scope of the present invention, administration of lactoferrin to subjects in need thereof is exclusively carried out by intravaginal topical route, i.e. in the use of the present invention lactoferrin is not administered to the subject by other routes, such as oral, intravenous or rectal intake, in addition to the intravaginal administration.

Within the scope of the present invention, the subject in need of lactoferrin may be a woman or a female mammal, such as a primate, a farm animal or a pet.
Figure 1 shows the cervico-vaginal IL-6 levels on day 30 in the study of the example: IL-6 levels were statistically lower in the lactoferrin-treated group than in the control group.
Figure 2 shows the cervical length measurements on day 30 in the study of the example: CL (cervical length) measurements were statistically lower in the control group than in the lactoferrin-treated group.

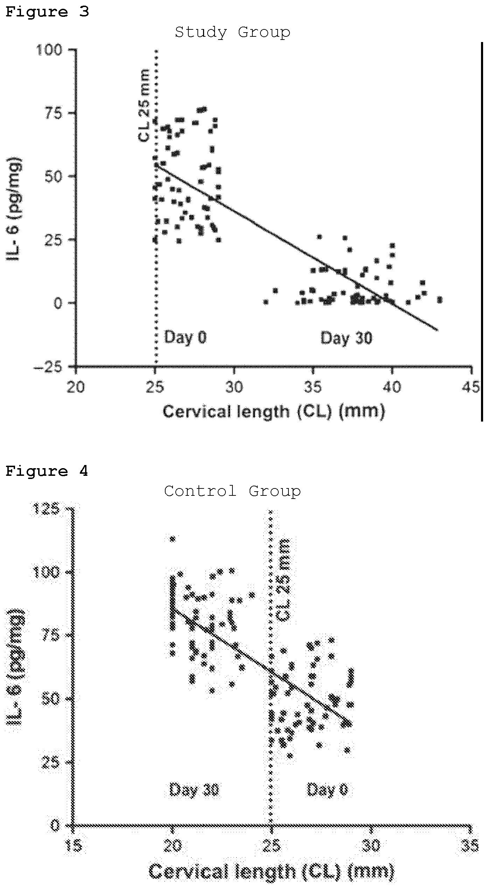

Figure 3 shows the cervical length measurements and IL-6 levels on day 0 and on day 30 in the study group (i.e. the group of subjects who did not receive a lactoferrin tablet) in the study of the example. The decrease in IL-6 levels is inversely related to an increase in CL measurements.
Figure 4 shows the cervical length measurements and IL-6 levels on day 0 and on day 30 in the control group (i.e. the group of subjects who did not receive a lactoferrin tablet) in the study of the example. The increase in IL-6 levels is inversely related to a decrease in CL measurements.

In one embodiment, the present invention relates to lactoferrin for use in the prevention and treatment of preterm delivery and miscarriage, wherein said use comprises administering a composition, wherein the amount of lactoferrin is from 250 to 800 mg per unit, once or twice a day by topical intravaginal administration to a subject in need thereof.

It was surprisingly found that the administration of high doses of lactoferrin once or twice a day is effective and practically free from side effects. This dosage regimen is in sharp contrast with the teachings of the prior art, wherein lactoferrin is administered intravaginally only in single relatively low quantities (e.g. 100 mg) and at intervals of 4 or 6 hours.

Arguably, the dosage followed in the prior art is based on the assumption that lactoferrin is almost completely metabolized and inactivated shortly after intake. Hence, following this assumption, to achieve an effective concentration of lactoferrin for an extended time, it would be necessary to repeat the administration to the subject at short time intervals.

The present inventor found, instead, that the administration can be carried out effectively by intake of higher doses and for one or two times a day, with significant advantages for the ease of the subject in need of lactoferrin and for the compliance with the prescribed dosage.

Preferably, lactoferrin for use according to the present invention is the complete protein of mammal origin. Preferably, lactoferrin for use according to the present invention is of bovine origin.

The composition for use in the present invention may comprise lactoferrin and at least one physiologically suitable excipient, i.e. at least a substance known to the person skilled in the art as suitable for use in a dosage form for intravaginal delivery of an active substance.

Preferably, the composition comprising lactoferrin for use according to the present invention is in the form of intravaginal tablet.

Preferably, in the composition according to the present invention the amount of lactoferrin for single intravaginal dosage form is 350, 400, 450, 500, 550, 600, 650, 700, 750 or 800 mg.

Preferably, in the composition according to the present invention, the amount of lactoferrin is between 600 and 800 mg for single intravaginal dosage form per day. Preferably, the composition for use according to the present invention is administered once a day and the amount of lactoferrin therein is 300 mg.

Preferably, the composition for use according to the present invention is administered once a day and the amount of lactoferrin therein is 800 mg.

Preferably, the composition for use according to the present invention is administered twice a day and in each administration the amount of lactoferrin is 250 mg. Preferably, the composition for use according to the present invention is administered twice a day and in each administration the amount of lactoferrin is 300 mg. Preferably, the composition for use according to the present invention is administered twice a day and in each administration the amount of lactoferrin is 250 mg.

As non-limiting examples, when the composition is administered twice per day, the second dosage form of the lactoferrin-comprising composition can be administered 4, 6, 8, 10 or 12 hours after the first administration of the day.

Preferably, when the composition is administered twice per day, most preferably in the above dosages, the second dosage form of the lactoferrin-comprising composition is administered 4 or 6 hours after the first administration. Preferably, when the composition is administered twice per day, most preferably in the above dosages, the second dosage form of the lactoferrin-comprising composition is administered 8 or 10 hours after the first administration.

Preferably, when the composition is administered twice per day, most preferably in the above dosages, the second dosage form of the lactoferrin-comprising composition is administered 12 hours after the first administration. Preferably, lactoferrin for use according to the present invention is administered as a prevention of miscarriage and / or preterm birth in high-risk pregnancies, one or two times a day for 7 days per month from the beginning of pregnancy up to the thirty-seventh week of pregnancy. According to the NIH (USA) guidelines, high-risk pregnancies are generally (but not exclusively) defined as pregnancies in the presence of at least one risk factor including high blood pressure, pre-eclampsia, eclampsia, polycystic ovary syndrome, diabetes, kidney diseases, autoimmune diseases, HIV/AIDS; obesity, teen pregnancy, first-time pregnancy after age 35, alcohol use, cigarette smoking, multiple gestation, gestational diabetes and the like.

Preferably, lactoferrin for use according to the present invention is administered during pregnancies in case of cervicometry values that are reduced with respect to those expected for the relevant gestational period or not corresponding to the gestational period, with or without contractions, once or twice a day from the twentieth week until the fortieth week.

For the scope of the present invention, cervicometry values are determined by objective examination of the uterine cervix, usually by ultrasound analysis, based on determining the length of the closed portion of the cervix and the shape of the internal os.

The assessment of cervicometry parameters in relation to the corresponding gestational period is accomplished according to the procedure known to those skilled in the art, for example, following the indications of Mella and Berghella (Sem. in Perinatol. 2009, 317-324), Mára M et al, Med Sci Monit, 2002; 8(5): MT72-77 and Berghella et al. Cochrane Database of Systematic Reviews 2009 Jul 8 ; (3): CD007235.

Preferably, lactoferrin for use according to the present invention is administered to subjects with normal blood levels of iron (serum iron levels normally above 50 µg/dL) and/or are free of blood coagulation diseases, i.e. to subjects which do not suffer chronically from diseases such as iron deficieny anemia or thrombophilia. In another aspect, the present invention comprises composition in a unit dosage form suitable for single intravaginal administration comprising at least one excipient and an amount of lactoferrin from 350 to 800 mg.

Preferably, the intravaginal dosage form for the composition according to the present invention is selected from a tablet, an ovule, a cream, a gel, a lipogel, a suppository or a capsule. More preferably, the intravaginal device according to the present invention is a tablet, an ovule or a capsule.

The composition of the present invention may comprise all excipients suitable for the production of solid or gel preparations for intravaginal use, as known by the person skilled in the art.

Preferably, in the composition according to the present invention the amount of lactoferrin is 350, 400, 450, 500, 550, 600, 650, 700, 750 or 800 mg for single intravaginal dosage form.

By way of non-limiting examples, such excipients may include diluents, absorbents, adsorbents, lubricants, ant-adherent additives, glidants, binders, disintegrants, surfactants, colorants, antioxidants, antimicrobials and polymers.

Preferably, in the composition according to the present invention the at least one excipient suitable for intravaginal administration is selected from mannitol, magnesium stearate, vegetable magnesium stearate, hydroxyethyl cellulose, crospovidone, polycarbophil, sodium carboxymethylcellulose and mixtures thereof. Preferably, the composition according to the present invention comprises lactoferrin, mannitol, magnesium stearate, hydroxyethylcellulose and polyethylene glycol. More preferably, in said composition the amount of lactoferrin is 350, 600 or 800 mg.

The composition according to the present invention can be prepared by all methods suitable and known to the expert of the branch.

The following example illustrates one embodiment of the invention, without limiting its scope.

### EXAMPLE

### Methods

### - Study participants

A randomised, prospective, longitudinal study was conducted from June 2009 to December 2010. A total of 3,324 patients who were undergoing routine ultrasonography at 20 - 24 weeks of gestational age for examination of fetal anatomy and growth, were given the option of transvaginal ultrasonographic measurements of cervical length as a predictor of spontaneous PTL. Women with CL 25 - 29 mm are invited to take part in a randomised treatment - no treatment trial of vaginal lactoferrin. The study is not conducted under double blind protocol.

Inclusion criteria are: patients at the first pregnancy (primigravida) at 20 - 24 weeks of gestation with singleton ongoing pregnancy, intact membranes and dating of pregnancy confirmed by 1st-trimester sonography. Exclusion criteria are: any known fetal, chromosomal or structural anomaly; ruptured membranes; intra-amniotic infections (based on clinical and biochemical parameters); vaginal bleeding; uterine contractions; known uterine anomalies; previous cervical disease; positive vaginal/urine culture; sexual intercourse and the need for an obstetrically indicated delivery.

The study was approved by the Institutional Review Board. A signed informed consent is obtained by all patients who join the study.

### - Randomisation and follow-up

The randomisation list is prepared using a computer-generated number list. Odds (treatment) and pairs (observation) defined treatment allocation. The list is managed by a senior midwife.

Women enrolled as study cases receive vaginal tablets comprising bovine lactoferrin (300 mg) as a single administration for 21 days (study group). Those randomised to observation received no drugs (control group). Both groups are similarly managed. Transvaginal ultrasound measurement (TVM) of the cervical length (CL) and cervico-vaginal washing for IL-6 assay are performed on the day of admission and after 30 days. Sonographic measurements of the cervical length are performed according to published guidelines and definitions (Mella and Berghella (Sem. in Perinatol. 2009, 317-324). CL 25 - 29 mm is assumed as a
short cervix (Ness et al. Am. J. Obstet. Gynecol. 2007, 197,:426.e1-e7)). TVM is performed by an experienced operator, who was blinded to the treatment allocation. Cervical fluid collection is performed by blinded operator. IL-6 analyses is carried out by another blinded operator. To assess infection, a vaginal swab and urine sample are collected, either at admission or at the follow-up day.

### - Sample collection and immunoassays

A total of 3 ml normal saline solution is used for cervico-vaginal washing after visualisation of the cervix by vaginal speculum.

The cervico-vaginal fluid is collected by a sterile pipette and transferred in sterile centrifuge tubes. The specimens are readily frozen in liquid nitrogen and stored at 80°C until analysis. For the analysis, the samples are thawed and centrifuged. To evaluate the concentration of secreted IL-6, supernatant is collected and stored at 4°C until use, while the pellet is suspended in lysis buffer and centrifuged to obtain the intracellular fraction of proteins. Before performing the assay, the intracellular concentration of proteins is determined by Eppendorf Biophotometer (Eppendorf GmbH, Hamburg, Germany) and expressed in µg/µl.

The Human IL-6 ELISA kits (Raybiotech Inc., GA, USA) ELHIL6-001 (specific for serum, plasma, cell culture supernatant and urine) and ELH-IL6 - 001C (specific for cell and tissue lysates) are employed to determine the concentration of secreted or intracellular fraction of IL-6, respectively. Of each sample, 200 µl are loaded in duplicate or triplicate and an enzyme linked immunosorbent
assay (ELISA) is performed following the manufacturer's protocol. Absorbance (optical density) is read with the Biotek ELx800 (Biotek, VT, USA) microplate reader at 450 nm.

ELISA kits employed in the study are suited for the detection of IL-6 in cell supernatant and lysed cells. Moreover, they are commercially available and validated by suppliers. Interleukin-6 levels are expressed as pg/mg of total proteins extracted from cervico-vaginal fluid.

### - Outcome measures

The primary outcome measures are the changes in the cervical length and in cervico-vaginal IL-6 levels. The secondary outcome measure is the tocographic detection of regular uterine contractions (more than 6 uterine contractions in 1 hour), pain and changes of the cervical consistency before the 37th week of gestation.

### - Statistical methods

The main outcome variables are cervico-vaginal IL-6 concentration and cervical length. The mean outcome is compared between the two randomised groups. The distributions of the variables are compared by Student's t-test. Means and standard deviations are used as descriptive statistics for each group.

Pearson's correlation coefficient (r) is used to evaluate the relationship, if any, between CL and IL-6 changes. The percentage of women with uterine contractions before the 37th week are compared using the χ² -test. Differences associated with p values of less than 0.05 are regarded as statistically significant. All statistical analyses are performed with professional statistic software (SPSS 7.5 for Windows).

### - Results

A total of 143 asymptomatic patients with a short cervix (CL:25 - 29 mm) could be enrolled; 113 had a CL less than 25 mm. They underwent standard management for patients at risk and were evaluated for the recovery room. A total of 221 had a CL 25 - 29 mm and of these, 163 were assessed for eligibility, homogeneous for age, race, lifestyle and inclusion criteria. A total of 143 women were then initially enrolled into the study.

Of the women, 71 were allocated to evaluate the effect of lactoferrin on cervical length and cervico-vaginal IL-6 concentration and allocated for treatment (study case group). The remaining 72 women were allocated for no treatment (control group).

A total of 15 women dropped out, as follows: seven in the study group (2 hydramnios; 2 rupture of membranes; 2 maternal hypertension; 1 abandoned the study) and eight in the control group (1 hydramnios; 3 rupture of membranes; 2 uterine contractions; 2 abandoned the study). Data from 128 cases are thus finally analysed for outcome.

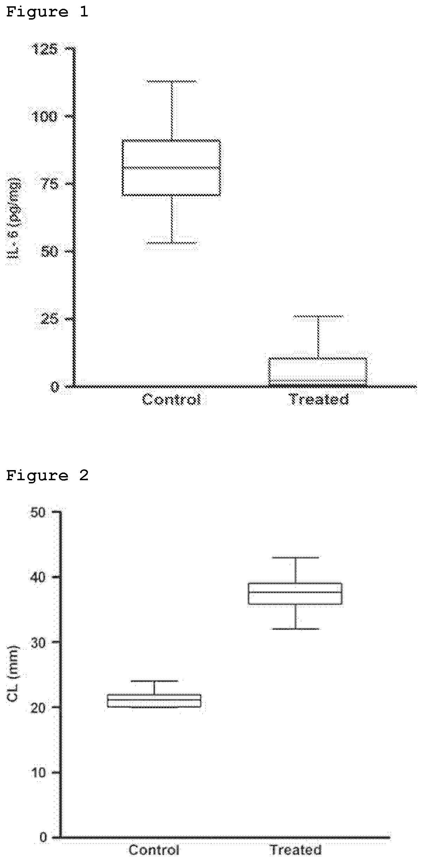

There is no significant difference in baseline IL-6 levels between the case and the control group (cases: mean 49.85 pg/mg; control: 49.59 pg/mg). No difference is found in cervical length between the two groups at the admission (control: mean: 27.1 mm; cases: mean 27.26 mm). On the day 30 evaluation, a statistical difference is found in cervical IL-6 levels between the two groups (cases: mean 5.823 pg/mg; range 0.1-12.81 pg/mg; controls: mean 80.82 pg/mg; range 55.8- 113.0 pg/mg) (p less than 0.0001) (Figure 1). The ELH-IL6 - 001 kit, specific for the detection of Il-6 cell culture supernatant, did not reveal the presence of the protein in supernatant, probably due to sample dilution. The intracellular fraction of Il-6 was easily detected and measured instead.

A difference is found in CL measurements between the two groups on day 30 follow-up (treated: mean CL: 37.6 mm; range 34.8 - 43.0 mm; controls: mean CL: 21.38 mm; range 20.0 - 23.5 mm) (*p* less than 0.0001) (Figure 2).

A negative relationship between CL measurements and IL-6 levels is found in both groups (cases: [*r* = - 0.81]; *p* less than 0.0001; controls: [r = - 0.73]; p less than 0.0001).

Five women (9%) out of the 64 in the lactoferrin group, and 13 women (20.3%) out of 64 in the control group, show more than six uterine contractions in 30 min, with changes in cervical length and softening of the cervix prior to 37 completed weeks' gestation (χ² = 4.14; p less than 0.05).

These subjects are submitted to standard protocols for PTD

### prevention.

These data show that high levels of intracellular inflammatory cytokine IL-6 appear related to a short cervix. A single daily administration of lactoferrine at high dosage (300 mg) effectively prevents miscarriage and pre-term delivery and is devoid of significant side effects.

## Claims

1. Lactoferrin for use in the prevention and treatment of preterm delivery and miscarriage, consisting of administering once or twice a day by topical intravaginal route to a subject in need thereof a unit dosage form, consisting of:
• lactoferrin in amounts of from 250 to 800 mg and
• at least one excipient suitable for intravaginal intake.

2. Lactoferrin for use according to claim 1 in which lactoferrin is bovine lactoferrin, or a fragment or derivative thereof.

3. Lactoferrin for use according to one of the preceding claims wherein the composition is in the form of an intravaginal tablet.

4. Lactoferrin for use according to one of the preceding claims wherein the composition is administered once a day and the amount of lactoferrin therein is 300 mg.

5. Lactoferrin for use according to one of the preceding claims, wherein, for use in preventing preterm birth and miscarriage in pregnancies at risk, lactoferrin is administered once or twice a day for seven days a month from the beginning of pregnancy up to the thirty-seventh week of pregnancy.

6. Lactoferrin for use according to one of the preceding claims, wherein, for use in preventing preterm birth and miscarriage in pregnancies in case of reduced cervicometry with respect to the normal value for the gestational period, or not corresponding to the gestational period, with or without contractions, lactoferrin is administered once or twice a day from the twentieth week up to the fortieth week.

7. Lactoferrin for use according to one of the preceding claims, wherein the topical administration of lactoferrin is carried out in subjects with normal blood levels of iron and/or free of blood coagulation diseases.

8. The lactoferrin for the use according to any one of the preceding claims wherein the amount of lactoferrin in said unit dosage form ranges from 350 to 800 mg.

9. The lactoferrin for the use according to claim 8 wherein the amount of lactoferrin in said unit dosage form is from 600 to 800 mg.

10. The lactoferrin for the use according to any one claims 1 to 9, wherein the at least one excipient suitable for intravaginal intake is selected from mannitol, magnesium stearate, magnesium stearate from vegetable sources, hydroxyethyl cellulose, crospovidone, polycarbophil, sodium carboxymethylcellulose, and mixtures thereof.

11. The lactoferrin for the use according to anyone of claims 1-10, wherein said unit dosage form is selected from : an intravaginal tablet, an intravaginal capsule or an intravaginal ovule.

## Patentansprüche

1. Lactoferrin für den Einsatz in der Prävention und Behandlung der Frühgeburt und der Fehlgeburt, der aus der Verabreichung einer Dosiereinheit an ein bedürftiges Subjekt ein- oder zweimal pro Tag durch topische intravaginale Route besteht, bestehend aus:
• Lactoferrin in Mengen von 250 bis 800 mg und
• mindestens einem für intravaginale Aufnahme geeigneten Hilfsstoff.

2. Lactoferrin für den Einsatz nach Anspruch 1, wobei Lactoferrin Rinder-Lactoferrin oder ein Fragment oder Derivat davon ist.

3. Lactoferrin für den Einsatz nach einem der vorangehenden Ansprüche, wobei die Zusammensetzung in Form einer intravaginalen Tablette ist.

4. Lactoferrin für den Einsatz nach einem der vorangehenden Ansprüche, wobei die Zusammensetzung einmal pro Tag verabreicht wird und die Menge der Lactoferrin darin 300 mg ist.

5. Lactoferrin für den Einsatz nach einem der vorangehenden Ansprüche, wobei Lactoferrin ein- oder zweimal pro Tag für sieben Tage im Monat vom Beginn der Schwangerschaft bis zur siebenunddreißigsten Schwangerschaftswoche für den Einsatz in der Prävention der Frühgeburt und der Fehlgeburt in Risikoschwangerschaften verabreicht wird.

6. Lactoferrin für den Einsatz nach einem der vorangehenden Ansprüche, wobei Lactoferrin ein- oder zweimal pro Tag von der zwanzigsten Woche ab bis zur vierzigsten Woche für den Einsatz in der Prävention der Frühgeburt und der Fehlgeburt in Schwangerschaften im Falle einer reduzierten Zervikometrie in Bezug auf den Normalwert für die Gestationszeit oder nicht entsprechend der Gestationszeit, mit oder ohne Kontraktionen, verabreicht wird.

7. Lactoferrin für den Einsatz nach einem der vorangehenden Ansprüche, wobei die topische Verabreichung von Lactoferrin bei Subjekten mit normalem Eisenspiegel im Blut und/oder frei von Blutgerinnungskrankheiten durchgeführt wird.

8. Lactoferrin für den Einsatz nach einem der vorangehenden Ansprüche, wobei die Menge an Lactoferrin in der vorgenannten Dosiereinheit im Bereich von 350 bis 800 mg liegt.

9. Lactoferrin für den Einsatz nach Anspruch 8, wobei die Menge an Lactoferrin in der vorgenannten Dosiereinheit im Bereich von 600 bis 800 mg liegt.

10. Lactoferrin für den Einsatz nach einem der Ansprüche 1 bis 9, wobei der mindestens einen für intravaginale Aufnahme geeigneten Hilfsstoff aus Mannitol, Magnesiumstearat, pflanzlichem Magnesiumstearat, Hydroxyethylcellulose, Crospovidon, Polycarbophil, Natriumcarboxymethylcellulose und Mischungen davon ausgewählt wird.

11. Lactoferrin für den Einsatz nach einem der Ansprüche 1 bis 10, wobei die vorgenannte Dosiereinheit aus einer intravaginalen Tablette, einer intravaginalen Kapsel oder einem intravaginalen Ovulum ausgewählt wird.

## Revendications

1. Lactoferrine pour l'utilisation dans la prévention et le traitement de l'accouchement prématuré et des fausses couches consistant en l'administration une ou deux fois par jour par voie intravaginale topique à un sujet qui en a besoin d'une unité de dosage composée de:
• lactoferrine dans des quantités de 250 à 800 mg et
• au moins un excipient approprié à la prise par voie intravaginale.

2. Lactoferrine pour l'utilisation selon la revendication 1, où la lactoferrine est lactoferrine bovine, ou l'un de ses fragments ou dérivés.

3. Lactoferrine pour utilisation selon l'une des revendications précédentes, où la composition est sous la forme d'une tablette intravaginale.

4. Lactoferrine pour l'utilisation selon l'une des revendications précédentes, où la composition est administrée une fois par jour et la quantité de lactoferrine est de 300 mg.

5. Lactoferrine pour l'utilisation selon l'une des revendications précédentes, où pour l'utilisation dans la prévention de l'accouchement prématuré et des fausses couches dans des grossesses à risque, la lactoferrine est administrée une ou deux fois par jour pendant sept jours par mois depuis le début de la grossesse jusqu'à la trente-septième semaine de grossesse.

6. Lactoferrine pour l'utilisation selon l'une des revendications précédentes, où pour l'utilisation dans la prévention de l'accouchement prématuré et des fausses couches en cas de réduction de la cervicométrie par rapport à la valeur normale pour la période gestationnelle, ou ne correspondant pas à la période gestationnelle, avec ou sans contractions, la lactoferrine est administrée une ou deux fois par jour à partir de la vingtième semaine jusqu'à la quarantième semaine.

7. Lactoferrine pour l'utilisation selon l'une des revendications précédentes, où l'administration topique de lactoferrine est effectuée chez des sujets ayant des niveaux de fer normaux dans le sang et/ou sans maladies de coagulation sanguine.

8. Lactoferrine pour l'utilisation selon l'une quelconque des revendications précédentes, où la quantité de lactoferrine dans ladite unité de dosage varie de 350 à 800 mg.

9. Lactoferrine pour l'utilisation selon la revendication 8, où la quantité de lactoferrine dans ladite unité de dosage varie de 600 à 800 mg.

10. Lactoferrine pour l'utilisation selon l'une quelconque des revendications 1-9, où l'au moins un excipient approprié à la prise par voie intravaginale est sélectionné parmi mannitol, stéarate de magnésium, stéarate de magnésium végétal, hydroxyéthylcellulose, crospovidone, carboxyméthylcellulose de sodium, polycarbophil et leurs mélanges.

11. Lactoferrine pour l'utilisation selon l'une quelconque des revendications 1-10, où l'unité de dosage est sélectionnée parmi une tablette intravaginale, une capsule intravaginale ou un ovule intravaginal.
